# FASCICULE DE BREVET EUROPEEN

(11) **EP 3 720 377 B1**
(45) Date de publication et mention de la délivrance du brevet: **25.09.2024**
(21) Numéro de dépôt: 18808378.6
(22) Date de dépôt: 03.12.2018
(51) Int. Cl.: A61B 17/88, A61B 17/17, A61B 90/11

(54) **ANCILLAIRE POUR CHIRURGIE OSSEUSE, AVANTAGEUSEMENT POUR ARTHROSCOPIE OPERATOIRE**
ZUBEHÖR FÜR KNOCHENCHIRURGIE, INSBESONDERE FÜR OPERATIVE ARTHROSKOPIE
ACCESSORY FOR BONE SURGERY, ADVANTAGEOUSLY FOR OPERATIVE ARTHROSCOPY

(30) Priorité: 05.12.2017 FR 1761676
(43) Date de publication de la demande: 14.10.2020
(73) Titulaire: Newclip International, 2163 Luxembourg (LU)
(72) Inventeur: LAMI, Damien, 83270 Saint Cyr Sur Mer (FR); GRILLO, Jean-Charles, 83110 Sanary Sur Mer (FR); PODGORSKI, Jean-Pierre, 49230 Sevremoine (FR); LARCHE, Grégoire, 49300 Cholet (FR)
(74) Mandataire: Jacobacci Coralis Harle
(86) Numéro de dépôt international: PCT/EP2018/083386
(87) Numéro de publication internationale: WO 2019/110531

(56) Documents cités:
- FR-A1- 2 996 114
- US-A1- 2009 318 923

## Description

### DOMAINE TECHNIQUE AUQUEL SE RAPPORTE L'INVENTION

La présente invention concerne, de manière générale, le domaine de la chirurgie osseuse.

Elle concerne en particulier les ancillaires pour chirurgie osseuse, avantageusement pour arthroscopie opératoire (par exemple pour une intervention de Latarjet), adaptés au positionnement et à la fixation d'un fragment osseux sur une surface osseuse cible.

### ARRIERE-PLAN TECHNOLOGIQUE

Certaines techniques de chirurgie osseuse préconisent de sectionner un fragment osseux, avant de le positionner et de le fixer sur une surface osseuse cible.

C'est par exemple le cas dans l'opération de Latarjet (ou « butée osseuse coracoïdienne ») qui consiste à venir mettre en place, en avant de l'épaule, un bloc osseux (coracoïde) et un tendon normalement fixé dessus (coraco-biceps).

Cette opération est généralement pratiquée chez les patients présentant des épaules qui se luxent, et dont les structures anatomiques abimées ne sont pas réparables ou ne sont pas suffisantes pour stabiliser l'épaule.

L'intervention de Latarjet « à ciel ouvert » est actuellement la technique standard.

Une réalisation sous arthroscopie a également été développée afin, notamment, de positionner la butée de façon plus précise et de réaliser si besoin, dans le même temps opératoire, un geste associé sur les parties molles.

Cependant, malgré la simplicité de son principe, les ancillaires actuels, décrits par exemple dans le document US-2009/0318923, sont sources de difficultés techniques qui peuvent conduire à des complications graves et qui nécessitent des temps d'intervention longs.

Compte tenu de ce qui précède, il existe un besoin de nouveaux ancillaires pour chirurgie osseuse, avantageusement pour arthroscopie opératoire, qui permettraient de concilier une intervention rapide et la moins traumatisante possible, tout en assurant un positionnement précis du fragment osseux puis une fixation solide sur la surface osseuse cible.

### OBJET DE L'INVENTION

Afin de remédier à l'inconvénient précité de l'état de la technique, la présente invention propose un ancillaire pour chirurgie osseuse, avantageusement pour arthroscopie opératoire (par exemple pour une intervention de Latarjet), adapté au positionnement et à la fixation d'un fragment osseux sur une surface osseuse cible.

Cet ancillaire comprend :
(i) au moins une poignée de manipulation,
(ii) un corps de visée comportant - deux faces frontales opposées, l'une avant et l'autre arrière, et - au moins un orifice de visée (par exemple un ou deux) qui débouche au niveau desdits deux faces frontales, pour le guidage d'au moins une vis et/ou broche destinée à être implantée dans ledit fragment osseux et dans ladite surface osseuse cible,
(iii) un organe palpeur, s'étendant en saillie de la face frontale avant dudit corps de visée et comportant une face inférieure destinée à venir prendre appui sur une partie de ladite surface osseuse cible.

La face frontale avant dudit corps de visée et la face inférieure dudit organe palpeur forment ensemble une surface de réception diédrique contre laquelle ledit fragment osseux est destiné à prendre appui.

Selon l'invention, ledit ancillaire comprend des moyens presseurs adaptés au maintien temporaire dudit fragment osseux en appui contre ladite surface de réception diédrique.

L'ancillaire pour chirurgie osseuse selon l'invention facilite ainsi le processus opératoire, notamment dans le cadre d'une intervention de Latarjet.

Cet ancillaire possède en particulier comme avantage de permettre un positionnement correct, de manière rapide et reproductible, du fragment osseux sur sa surface osseuse cible.

Selon un mode de réalisation préféré, les moyens presseurs comprennent :
- un panneau d'appui agencé en regard de la face frontale avant dudit corps de visée, et
- des moyens de guidage en translation, adaptés au guidage en translation dudit panneau d'appui selon un axe de translation orienté parallèlement audit au moins un orifice de visée du corps de visée de sorte à assurer son réglage en écartement par rapport à ladite face frontale avant du corps de visée.

D'autres caractéristiques non limitatives et avantageuses de ce mode de réalisation préféré, prises individuellement ou selon toutes les combinaisons techniquement possibles, sont les suivantes :
- le panneau d'appui des moyens presseurs comporte au moins une réservation ménagée en regard dudit au moins un orifice de visée équipant le corps de visée ;
- les moyens presseurs coopèrent avec ledit corps de visée par le biais desdits moyens de guidage en translation ; les moyens presseurs et ledit corps de visée coopèrent par le biais d'au moins un couple nervure / rainure ; le corps de visée comporte de préférence deux faces latérales comportant chacune un premier élément dudit couple nervure / rainure, et les moyens presseurs comportent une embase qui porte le panneau d'appui et qui est munie d'un second élément dudit couple nervure / rainure ; encore de préférence, l'embase des moyens presseurs comporte un panneau inférieur, en regard d'une face inférieure dudit corps de visée, lequel panneau inférieur comporte deux extrémités : une première extrémité munie du panneau d'appui, et une seconde extrémité munie des moyens de guidage en translation ;
- les moyens presseurs comportent des moyens de préhension, avantageusement portés par ladite embase, pour la manoeuvre en translation par un opérateur dudit panneau d'appui ; les moyens de préhension desdits moyens presseurs comprennent avantageusement deux crochets latéraux qui s'étendent de part et d'autre du corps de visée et qui s'ouvrent en regard dudit panneau d'appui ;
- les moyens de guidage en translation comportent des moyens d'indexation (par exemple des dentures), pour conférer un pas en translation audit panneau d'appui, par exemple un pas compris entre 1 et 3 mm ; les moyens presseurs comportent avantageusement des moyens pour inactiver lesdits moyens d'indexation, en particulier pour une manoeuvre en écartement dudit panneau d'appui par rapport à la face frontale avant dudit corps de visée.

D'autres caractéristiques non limitatives et avantageuses de l'ancillaire conforme à l'invention, prises individuellement ou selon toutes les combinaisons techniquement possibles, sont les suivantes :
- les moyens presseurs sont amovibles par rapport audit ancillaire ;
- l'organe palpeur coopère avec ledit corps de visée par le biais de moyens de réglage en hauteur, selon un second axe de translation orienté perpendiculairement à l'axe dudit au moins un orifice de visée ; l'organe palpeur porte avantageusement une graduation métrique orientée selon ledit second axe de translation ;
- le corps de visée comporte deux orifices traversants juxtaposés et coplanaires dans un plan de visée ; ladite au moins une poignée s'étend avantageusement dans un plan perpendiculaire, ou au moins approximativement perpendiculaire, audit plan de visée.

L'invention propose également un système d'ancillaires, adapté à être couplé à une arthroscopie, comprenant :
(i) au moins un premier ancillaire selon l'invention,
(ii) au moins un second ancillaire du type visserie,
lequel au moins un second ancillaire comprend :
- une portion amont, destinée à pénétrer dans ledit orifice de visée du corps de visée et à faire saillie au niveau de ladite face frontale avant, qui comporte une extrémité amont formant tête de forage / vissage et un profil de filet progressif, et
- une portion aval, destinée à coopérer avec un organe de manoeuvre en rotation,
- une structure de butée ménagée entre lesdites portions amont et aval, adaptée à venir en butée contre la face frontale arrière du corps de visée, et
- un canal central, agencé coaxialement audit second ancillaire, adapté au passage d'une broche.

De préférence, le système d'ancillaires comprend deux seconds ancillaires ayant des portions aval de longueurs différentes l'une par rapport à l'autre.

Le système d'ancillaires comprend encore avantageusement une réglette comportant :
- une première partie de mesure munie d'une graduation, adaptée à mesurer la profondeur d'enfoncement d'une broche dans le fragment osseux et la surface osseuse cible,
- une seconde partie de mesure munie d'une graduation, adaptée à mesurer la largeur du fragment osseux, et
- une partie d'assemblage avec le corps de visée, au niveau de sa face frontale arrière.

L'invention propose aussi un procédé de chirurgie osseuse, avantageusement hybride « ciel ouvert / arthroscopie », comprenant :
- une mesure de la largeur du fragment osseux, avantageusement à l'aide de ladite de la réglette,
- un réglage de la hauteur de l'organe palpeur du premier ancillaire tenant compte de la mesure obtenue avec la réglette,
- un maintien du fragment osseux contre la surface d'appui diédrique du premier ancillaire, grâce aux moyens presseurs,
- une insertion d'au moins un second ancillaire au travers dudit au moins un orifice de visée équipant le corps de visée et dans ledit fragment osseux,
- un retrait des moyens presseurs,
- un positionnement du fragment osseux contre la surface osseuse cible,
- une insertion d'au moins une broche au travers dudit au moins un second ancillaire,
- une mesure de la profondeur d'insertion de ladite au moins une broche, au moyen de la réglette, pour déterminer la longueur de ladite au moins une vis de compression à poser,
- un retrait successivement de chaque second ancillaire et son remplacement par ladite vis de compression.

### DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 est une vue générale et en perspective d'un ancillaire pour chirurgie osseuse, avantageusement pour arthroscopie opératoire (par exemple pour une intervention de Latarjet),
- la figure 2 est une vue partielle et agrandie de l'ancillaire selon la figure 1, vue de côté ;
- la figure 3 est une vue générale et en perspective de l'ancillaire selon les figures 1 et 2, représenté ici de manière éclatée ;
- la figure 4 représente un système d'ancillaires selon l'invention, comprenant notamment l'ancillaire selon les figures 1 à 3 ;
- les figures 5 et 6 représentent des ancillaires du type préhenseur/tournevis, partie du système d'ancillaires selon la figure 4 ;
- la figure 7 est une vue de dessus d'une réglette constitutive du système d'ancillaires selon la figure 4 ;
- la figure 8 montre la mise en oeuvre du système d'ancillaires selon l'invention, au cours d'une intervention de chirurgie osseuse de Latarjet.

### Ancillaire pour chirurgie osseuse

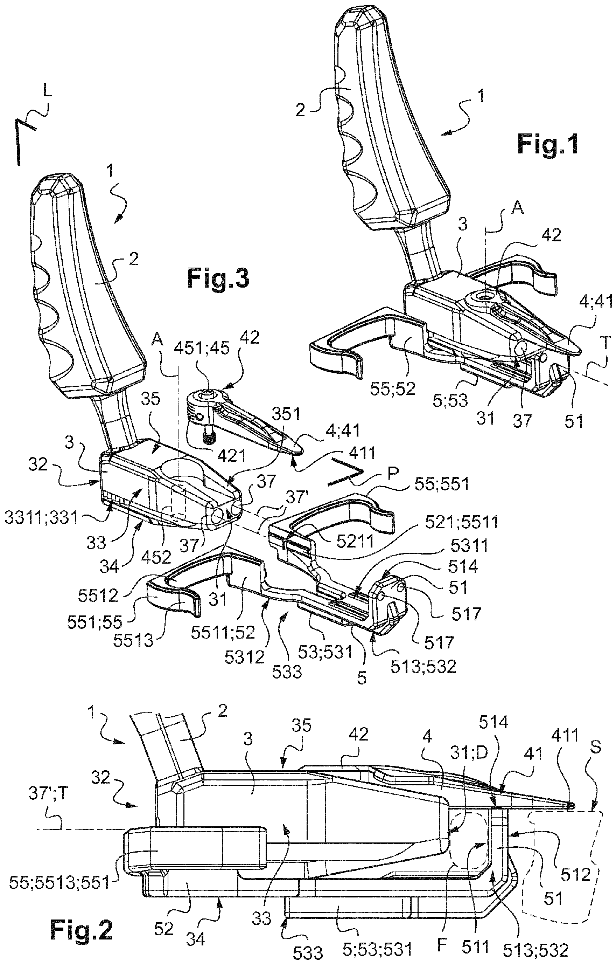

Les figures 1 à 3 représentent un ancillaire 1 selon l'invention qui est adapté pour la chirurgie osseuse, avantageusement pour arthroscopie opératoire.

L'arthroscopie opératoire permet des interventions chirurgicales intra-articulaires, mini-invasives.

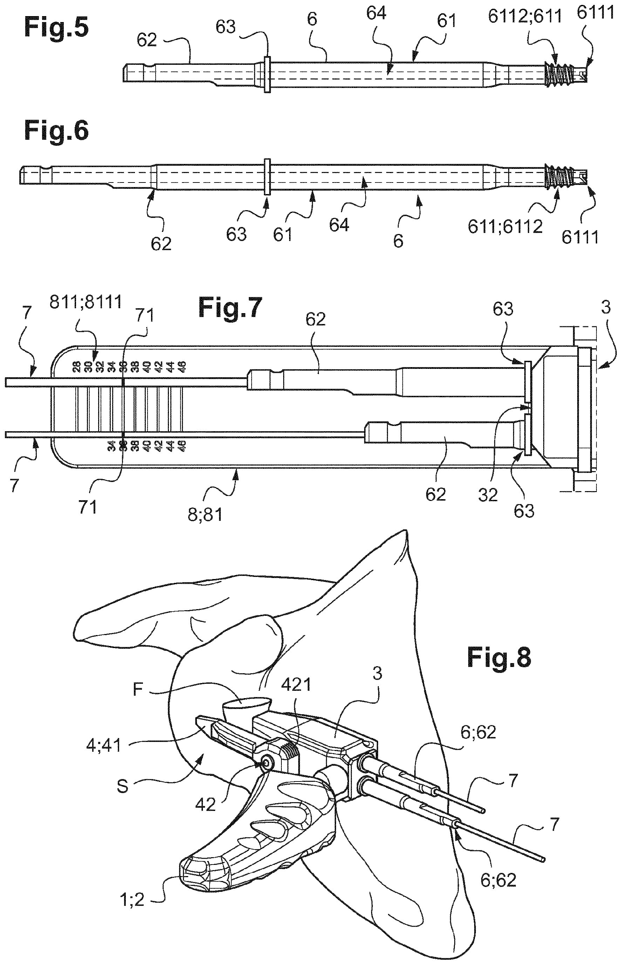

En particulier, l'ancillaire 1 selon l'invention est adapté au positionnement et à la fixation d'un fragment osseux F sur une surface osseuse cible S (figure 8).

Un tel ancillaire 1 est particulièrement adapté à la réalisation d'une intervention de Latarjet.

Cette intervention de Latarjet consiste en une opération chirurgicale au cours de laquelle le fragment osseux F est utilisé comme une butée osseuse destinée à être positionnée au niveau de la partie antérieure de la glène ; cette butée osseuse est réalisée à partir de l'apophyse coracoïde.

L'ancillaire 1 selon l'invention, dit encore « ancillaire de visée » ou « guide de visée », comprend pour cela différentes parties :
- une poignée de manipulation 2,
- un corps de visée 3, pour le guidage d'au moins une vis et/ou broche destinée à être implantée dans le fragment osseux F (par exemple l'apophyse coracoïde) et/ou dans la surface osseuse cible S (par exemple la partie antérieure de la glène),
- un organe palpeur 4, destinée à venir prendre appui sur une partie de la surface osseuse cible S, et
- des moyens presseurs 5 comportant notamment un panneau d'appui 51, adapté au maintien temporaire du fragment osseux F au sein de l'ancillaire 1.

Le corps de visée 3 se présente ici sous la forme d'un bloc de forme générale parallélépipédique.

Ce corps de visée 3 est délimité par un ensemble de faces (figures 2 et 3) :
- deux faces frontales opposées, l'une avant 31 et l'autre arrière 32,
- deux faces latérales 33, opposées,
- une face 34 dite « inférieure », et
- une face 35 dite « supérieure ».

Ce corps de visée 3 comporte au moins un orifice de visée 37, ici au nombre de deux (figure 3). Chaque orifice de visée 37 est adapté au guidage d'au moins une vis et/ou une broche destinée à être implantée dans le fragment osseux F et/ou dans la surface osseuse cible S.

Chaque orifice de visée 37 est ici traversant : il débouche au niveau des deux faces frontales 31, 32 précitées.

Les orifices de visée 37 définissent chacun un axe longitudinal 37'. Les axes longitudinaux 37' s'étendent ici parallèlement l'un par rapport à l'autre, de manière juxtaposée et coplanaire dans un plan général P qui est encore désigné sous le nom de « plan de visée » (figure 3).

Les deux faces latérales 33 comportent ici des moyens de guidage en translation 331 qui sont destiné à coopérer avec les moyens presseurs 5 pour le guidage en translation de ces derniers.

Ces moyens de guidage en translation 331 comportent ici un premier élément 331 d'un couple nervure / rainure, en l'espèce une rainure s'étendant parallèlement à l'axe longitudinal 37' des orifices de visée 37 (figure 3).

Cette rainure 331 comporte ici des moyens d'indexation, par exemple une denture 3311 (plusieurs dents réparties sur la longueur de la rainure 331), pour conférer un pas en translation au panneau d'appui 51. Ce pas en translation est par exemple compris entre 1 et 3 mm.

Comme décrit par la suite, un tel pas en translation est intéressant pour assurer un maintien amovible du réglage en écartement appliqué au panneau d'appui 51 lors du pincement du fragment osseux F.

L'organe palpeur 4 comporte deux parties :
- une partie avant 41, s'étendant en saillie de la face frontale avant 31 du corps de visée 3, et
- une partie arrière 42, disposée au niveau de la face supérieure 35 du corps de visée 3 pour sa fixation amovible avec ce dernier.

La partie arrière 42 de l'organe palpeur 4 est ici rapportée au sein d'un logement 351 (ou empreinte) qui est ménagé dans la face supérieure 35 du corps de visée 3 (figure 3).

Cette partie arrière 42 de l'organe palpeur 4 coopère avec le corps de visée 3 par le biais de moyens de réglage en hauteur 45, selon un axe de translation A orienté perpendiculairement à l'axe longitudinal 37' des orifices de visée 37.

La partie arrière 42 de l'organe palpeur 4 est guidée en translation par le logement 351 complémentaire.

Pour cela, la partie arrière 42 de l'organe palpeur 4 porte une vis 451 (avec un degré de liberté en rotation et sans degré de liberté en translation) qui coopère avec un orifice taraudé 452 (représenté schématiquement sur la figure 3) orienté coaxialement par rapport audit axe de translation A.

La course en hauteur de cet organe palpeur 4 va par exemple de 0 mm à 6 mm, correspondant à un décalage allant de 3,5 à 9,5 mm.

La partie arrière 42 de l'organe palpeur 4 porte encore une graduation métrique 421 qui est orientée selon l'axe de translation A précité.

Cette graduation métrique 421 est destinée à servir de repère pour le réglage en hauteur de l'organe palpeur 4, tenant compte des dimensions du fragment osseux F.

Cette graduation métrique 421 est agencée sur la partie arrière 42, latéralement et sur sa hauteur, de sorte à utiliser la face supérieure 35 du corps de visée 3 comme repère de réglage en hauteur.

La partie avant 41 de cet organe palpeur 4 comporte quant à elle une face inférieure 411 qui est destinée, tel que décrit par la suite, à venir prendre appui simultanément sur le fragment osseux F et sur une partie de la surface osseuse cible S.

Cette face inférieure 411 de l'organe palpeur 4 forme, en combinaison avec la face frontale avant 31 du corps de visée 3, une surface de réception diédrique D contre laquelle le fragment osseux F est destiné à prendre appui et à être maintenu par les moyens presseurs 5 (figure 2).

Les moyens presseurs 5 sont adaptés au maintien temporaire du fragment osseux F en appui contre cette surface de réception diédrique D (figure 2). Ces moyens presseurs 5 sont en outre avantageusement amovibles par rapport à l'ancillaire 1, utile lors du positionnement et de la fixation du fragment osseux F.

Ces moyens presseurs 5 comprennent en l'espèce :
- le panneau d'appui 51 agencé en regard de la face frontale avant 31 du corps de visée 3,
- des moyens de guidage en translation 52, adaptés au guidage en translation de ce panneau d'appui 51 selon un axe de translation T (figure 2) orienté parallèlement à l'axe longitudinal 37' des orifices de visée 37 du corps de visée 3, et
- une embase 53 portant ce panneau d'appui 51 et ces moyens de guidage en translation 52.

L'embase 53 comporte ici un panneau inférieur 531, destiné à venir en regard de la face inférieure 34 du corps de visée 3.

Ce panneau inférieur 531 comporte deux extrémités :
- une première extrémité 532, avant, munie du panneau d'appui 51, et
- une seconde extrémité 533, arrière, munie des moyens de guidage en translation 52.

Ce panneau inférieur 531 présente ici une forme générale en Y, ou de fourche (figure 3), comprenant :
- un bras avant 5311 portant le panneau d'appui 51, et
- deux branches arrière 5312 portant les moyens de guidage en translation 52.

Cette forme de réalisation de l'embase 53 vise à autoriser une déformation élastique en écartement des branches arrière 5312 et des moyens de guidage en translation 52 associés.

Le panneau d'appui 51 comporte différentes parties (figures 2 et 3) :
- une face arrière 511, orientée vers la face frontale avant 31 du corps de visée 3,
- une face avant 512, opposée à ladite face arrière 511,
- une bordure inférieure 513, solidarisée avec l'embase 53, et
- une bordure supérieure 514, libre, destinée à venir en regard et à cheminer le long de la face inférieure 411 de l'organe palpeur 4.

Le panneau d'appui 51, et en particulier sa face arrière 511, comporte au moins une réservation 517 (borgne ou traversante) ménagée en regard de chaque orifice de visée 37 équipant le corps de visée 3.

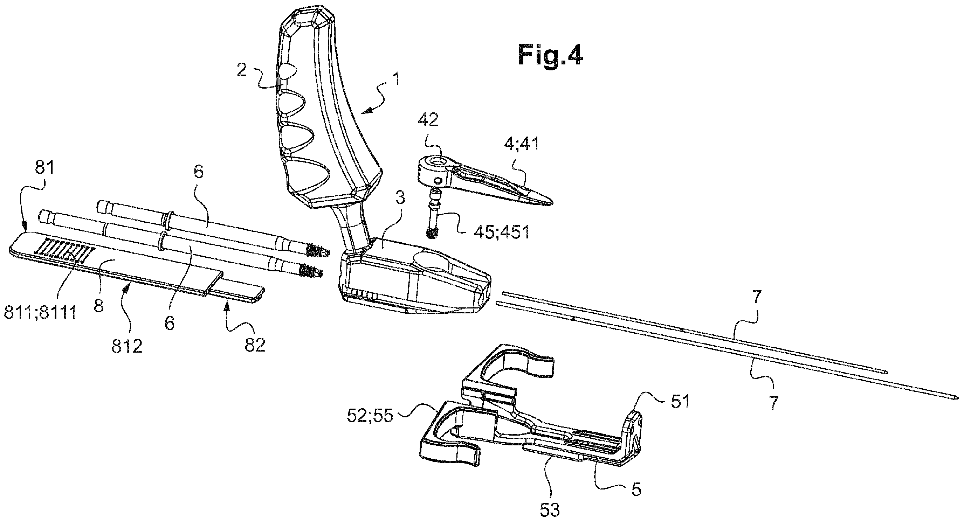

Chaque réservation 517 est utile pour recevoir l'extrémité libre d'un ancillaire rapporté au travers de l'orifice de visée 37 coaxial (en particulier un second ancillaire 6 décrit par la suite en relation avec la figure 4).

Les moyens de guidage en translation 52 autorisent le réglage en écartement du panneau d'appui 51 par rapport à la face frontale avant 31 du corps de visée 3 ; en d'autres termes, les moyens de guidage en translation 52 permettent un guidage en translation du panneau d'appui 51, parallèlement à lui-même, le long de l'axe de translation T.

Ces moyens de guidage en translation 52 autorisent en outre ici une séparation des moyens presseurs 5 par rapport au corps de visée 3, par un mouvement en extraction du côté de la face frontale avant 31 du corps de visée 3.

Pour cela, les moyens de guidage en translation 52 des moyens presseurs 5 coopèrent ici avec les moyens de guidage en translation 331 du corps de visée 3.

Ces moyens de guidage en translation 52 comprennent, en l'espèce, un second élément 521 du couple nervure / rainure adapté à coopérer, au jeu près, avec le premier élément 331 du couple nervure / rainure du corps de visée 3 (figure 3).

Le second élément 521 du couple nervure / rainure, ici une nervure, comporte des moyens d'indexation, par exemple au moins une dent 5211, destinés à coopérer par déformation élastique avec les moyens d'indexation 3311 de la rainure 331 équipant le corps de visée 3.

Les moyens presseurs 5 comportent encore des moyens de préhension 55, portés ici par l'extrémité arrière 533 de l'embase 53 et par les moyens de guidage en translation 52.

Les moyens de préhension 55 sont adaptés à la manoeuvre en translation par un opérateur des moyens presseurs 5 et de son panneau d'appui 51, en particulier dans un sens de rapprochement du panneau d'appui 51 vers la face frontale avant 31 du corps de visée 3.

Les moyens de préhension 55 comprennent pour cela deux crochets latéraux 551, ici en forme générale de U, qui s'étendent de part et d'autre du corps de visée 3 (ici symétriquement) et qui s'ouvrent en regard du panneau d'appui 51.

En l'espèce, chaque crochet latéral 551 comprend :
- un tronçon intérieur 5511, destiné à venir s'appuyer sur l'une des faces latérales 33 du corps de visée 3 et muni du second élément 521 du couple nervure / rainure,
- un tronçon transversal 5512, servant de surface d'appui en traction pour l'opérateur, et
- un tronçon extérieur 5513, en regard et à distance du tronçon intérieur 5511.

Les deux tronçons intérieurs 5511 des crochets latéraux 551 s'étendent ainsi en regard et en distance l'un de l'autre. Ils sont destinés à prendre en sandwich les faces latérales 33 du corps de visée 3.

Les moyens presseurs 5 comportent encore ici, de manière optionnelle, des moyens pour inactiver les moyens d'indexation 3311, 5211, en particulier pour une manoeuvre libre en écartement du panneau d'appui 51 par rapport à la face frontale avant 31 du corps de visée 3 lors du démontage des moyens presseurs 5.

Ces moyens pour inactiver les moyens d'indexation 3311, 5211 sont ici formés par le tronçon extérieur 5513 des crochets latéraux 551.

En effet, une force de pincement visant à rapprocher les deux tronçons extérieurs 5513 tend à écarter et à faire diverger les tronçons intérieurs 5511 des crochets latéraux 551 par un phénomène de déformation élastique, conduisant alors à l'écartement des moyens d'indexation 5211 des nervures 521 par rapport aux moyens d'indexation 3311 des rainures 331. Les moyens presseurs 5 sont alors libres en coulissement sur la longueur du corps de visée 3, ce qui est en particulier utile pour séparer ces moyens presseurs 5 par rapport à l'ancillaire 1.

Pour être complet, la poignée 2 est ici solidarisée avec la face supérieure 35 du corps de visée 3, du côté de la face frontale arrière 32.

Cette poignée 2 s'étend avantageusement dans un plan L. perpendiculaire, ou au moins approximativement perpendiculaire, au plan de visée P.

En pratique, cet agencement de la poignée 2 s'avère particulièrement ergonomique pour le praticien, au cours du processus opératoire.

### Système d'ancillaires

L'ancillaire 1 selon l'invention, décrit ci-dessus en relation avec les figures 1 à 3, fait avantageusement partie d'un système d'ancillaires, adapté à être couplé à une arthroscopie, qui est représenté schématiquement sur la figure 4.

Ce système d'ancillaires comprend :
- au moins un premier ancillaire 1 décrit ci-dessus en relation avec les figures 1 à 3,
- au second ancillaire 6 du type visserie, ayant ici une fonction préhenseur et tournevis,
- au moins une broche 7,
- des vis de compression (non représentées), de préférence des vis de compression canulées, et avantageusement
- une réglette 8.

Les seconds ancillaires 6 du type visserie, représentés en détails sur les figures 5 et 6, sont notamment utiles pour le maintien temporaire du fragment osseux F sur l'ancillaire 1 après démontage des moyens presseurs 5.

Ces seconds ancillaires 6 comprennent :
- une portion amont 61, destinée à pénétrer dans l'orifice de visée 37 du corps de visée 3 et à faire saillie au niveau de la face frontale avant 31,
- une portion aval 62, destinée à coopérer avec un organe de manoeuvre en rotation (non représenté) qui a avantageusement la forme d'une poignée de tournevis,
- une structure de butée 63 ménagée entre les portions amont 61 et aval 62, adaptée à venir en butée contre la face frontale arrière 32 du corps de visée 3, et
- un canal central 64, agencé coaxialement au corps de ce second ancillaire 6, adapté au passage de la broche 7 précitée (comme illustré schématiquement sur la figure 7).

La portion amont 61 comporte un diamètre correspondant, au jeu près, au diamètre de l'orifice de visée 37 du corps de visée 3.

Cette portion amont 61 comporte une extrémité amont 611 qui se compose de deux parties en série :
- une tête de forage / vissage 6111, terminal, et
- un profil de filet progressif 6112.

La tête de forage / vissage 6111 présente une structure possédant deux fonctions :
- une fonction de tête de forage dans le fragment osseux F rapporté contre la surface de réception diédrique D, et
- une fonction de tête de vissage pour une tête d'une vis de compression destinée à être implantée entre le fragment osseux F et la surface osseuse cible S.

Le profil de filet progressif 6112 est quant à lui adapté à être vissé dans le fragment osseux F.

Les seconds ancillaires 6 sont avantageusement au nombre de deux et ont avantageusement des portions aval 62 qui sont de longueurs différentes l'une par rapport à l'autre. Cette différence de longueur vise à éviter un encombrement conflictuel lors du processus opératoire.

La réglette 8, en forme générale de platine, comporte avantageusement les parties suivantes :
- une partie arrière 81 adaptée à la mise en oeuvre de différentes mesures, et
- une partie avant 82 adaptée à l'assemblage avec le corps de visée 3, au niveau de sa face frontale arrière 32.

La partie arrière 81 comporte deux faces munies de parties de mesure :
- une face supérieure 811 formant une première partie de mesure munie d'une graduation métrique 8111 adaptée à mesurer la profondeur d'enfoncement d'une broche 7 dans le fragment osseux F et la surface osseuse cible S, et
- une face inférieure 812 formant une seconde partie de mesure munie d'une graduation métrique (non représentée), adaptée à mesurer la largeur du fragment osseux F.

La partie avant 82 forme quant à elle une partie d'un ensemble tenon / mortaise.

Cette partie avant 82 présente ici une forme de tenon destinée à venir se loger dans un logement complémentaire (non représenté) débouchant du côté de la face frontale arrière 32 du corps de visée 3.

La réglette 8 est ainsi adaptée à être montée sur le corps de visée 3, en saillie de sa face frontale arrière 32, de sorte à s'étendre en regard d'un tronçon de broches 7 débouchant du côté de cette face frontale arrière 32.

Les broches 7 sont destinées à permettre le méchage guidé et la validation du centrage et de l'entraxe des futures vis de compression.

Ces broches 7 comportent avantageusement un repère visuel 71 pour déterminer leur profondeur d'enfoncement à l'aide de la graduation métrique 8111 de la première partie de mesure 811 (figure 7).

La graduation métrique 8111 de la première partie de mesure 811 est ajustée en fonction de la longueur des broches 7 et de la position de son repère visuel 71.

En pratique, la profondeur d'enfoncement de chaque broche 7 est obtenue en relevant la valeur de la graduation métrique 8111 qui se situe en regard du repère visuel 71 de cette broche 7.

### Procédé

Le système d'ancillaires selon l'invention permet de concilier une intervention la moins traumatisante possible, tout en assurant un positionnement rapide et précis du fragment osseux puis une fixation solide sur la surface osseuse cible.

En pratique, tout d'abord, le procédé de chirurgie osseuse comprend un prélèvement et une préparation, selon une procédure classique, du fragment osseux F, par exemple le bloc osseux coracoïde (avec le tendon coraco-biceps) lors d'une intervention de Latarjet.

Le procédé de chirurgie osseuse comprend ensuite la succession des étapes suivantes, pour la préparation du premier ancillaire 1 et la solidarisation premier ancillaire 1 / fragment osseux F, à savoir :
- une mesure de la largeur du fragment osseux F, de préférence dans ses trois plans, avantageusement à l'aide de la réglette 8 (plus précisément au moyen de la seconde partie de mesure 812),
- un réglage de la hauteur de l'organe palpeur 4 du premier ancillaire 1 tenant compte de la mesure obtenue avec la réglette 8 sur le plan axial,
- une insertion du fragment osseux F dans le premier ancillaire 1 et la manoeuvre des moyens presseurs 5 de sorte que son panneau d'appui 51 maintient ce fragment osseux F par pincement avec la surface d'appui diédrique D du premier ancillaire 1,
- une insertion des seconds ancillaires 6 au travers des orifices de visée 37 équipant le corps de visée 3 depuis sa face frontale arrière 32, de sorte que l'extrémité amont 611 de chaque second ancillaire 6 débouche du côté de la face frontale avant 31 et soit vissée dans le fragment osseux F, générant une solidarisation temporaire premier ancillaire 1 / fragment osseux F (avantageusement le second ancillaire 6 le plus court est inséré en premier pour éviter les problèmes d'encombrement),
- un retrait des moyens presseurs 5 par rapport au premier ancillaire 1, de sorte que le fragment osseux F soit maintenu contre la surface d'appui diédrique D du premier ancillaire 1 par le biais des seconds ancillaires 6 et de sorte que le fragment osseux F soit libéré pour sa pose.

Ces différentes étapes préalables (prélèvement / préparation / fixation) sont avantageusement réalisées au cours d'un temps ouvert.

Le procédé de chirurgie osseuse comprend ensuite la succession des étapes suivantes pour le positionnement et la fixation du fragment osseux F contre la surface osseuse cible S, avantageusement au cours d'un temps arthroscopique, à savoir :
- un positionnement du fragment osseux F contre la surface osseuse cible S, guidé notamment par l'organe palpeur 4 qui vient en appui sur une partie de la surface osseuse cible S, par exemple la surface articulaire de la glène dans une intervention de Latarjet (figure 8),
- une insertion des broches 7 au sein des seconds ancillaires 6 de sorte qu'elles traversent chacune le fragment osseux F et la surface osseuse cible S (figure 8),
- une mesure de la profondeur d'insertion des broches 7 au moyen de la réglette 8 (plus précisément au moyen de la première partie de mesure 811), pour déterminer la longueur des vis de compression à poser (figure 7),
- un retrait successif de chaque second ancillaire 6 et son remplacement par une vis de compression qui est guidée au travers de chaque orifice de visée 37, sur la longueur d'une broche 7, et qui est vissée à l'aide de ce second ancillaire 6.

Le fragment osseux F est ainsi fixé sur la surface osseuse cible S par le biais des vis de compression qui traversent ce fragment osseux F et qui sont ancrées au niveau de la surface osseuse cible S.

Une fois le fragment osseux F convenablement fixé, les broches 7 et le premier ancillaire 1 peuvent être retirés.

Le procédé chirurgical est ainsi avantageusement hybride « ciel ouvert / arthroscopie », comprenant un prélèvement et une préparation de la butée au cours d'un temps ouvert puis le positionnement et la fixation du fragment osseux F contre la surface osseuse cible S au cours d'un temps arthroscopique.

## Revendications

1. Ancillaire pour chirurgie osseuse, avantageusement pour arthroscopie opératoire, adapté au positionnement et à la fixation d'un fragment osseux sur une surface osseuse cible,
lequel ancillaire (1) comprend :
(i) au moins une poignée de manipulation (2),
(ii) un corps de visée (3) comportant - deux faces frontales opposées, l'une avant (31) et l'autre arrière (32), et - au moins un orifice de visée (37) qui débouche au niveau desdits deux faces frontales (31, 32), pour le guidage d'au moins une vis et/ou broche destinée à être implantée dans ledit fragment osseux (F) et dans ladite surface osseuse cible (S),
(iii) un organe palpeur (4), s'étendant en saillie de la face frontale avant (31) dudit corps de visée (3) et comportant une face inférieure (411) destinée à venir prendre appui sur une partie de ladite surface osseuse cible (S),
laquelle face frontale avant (31) dudit corps de visée (3) et laquelle face inférieure (411) dudit organe palpeur (4) forment ensemble une surface de réception diédrique (D) contre laquelle ledit fragment osseux (F) est destiné à prendre appui,
**caractérisé en ce que** ledit ancillaire (1) comprend des moyens presseurs (5) adaptés au maintien temporaire dudit fragment osseux (F) en appui contre ladite surface de réception diédrique (D).

2. Ancillaire pour chirurgie osseuse, selon la revendication 1, **caractérisé en ce que** les moyens presseurs (5) comprennent :
- un panneau d'appui (51) agencé en regard de la face frontale avant (31) dudit corps de visée (3), et
- des moyens de guidage en translation (52), adaptés au guidage en translation dudit panneau d'appui (51) selon un axe de translation (T) orienté parallèlement audit au moins un orifice de visée (37) du corps de visée (3) de sorte à assurer son réglage en écartement par rapport à ladite face frontale avant (31) du corps de visée (3).

3. Ancillaire pour chirurgie osseuse, selon la revendication 2, **caractérisé en ce que** les moyens de guidage en translation (52) des moyens presseurs (5) coopèrent avec des moyens de guidage en translation (331) dudit corps de visée (3).

4. Ancillaire pour chirurgie osseuse, selon la revendication 3, **caractérisé en ce que** les moyens presseurs (5) et ledit corps de visée (3) coopèrent par le biais d'au moins un couple nervure / rainure (331, 521).

5. Ancillaire pour chirurgie osseuse, selon la revendication 4, **caractérisé en ce que** le corps de visée (3) comporte deux faces latérales (33) comportant chacune un premier élément (331) dudit couple nervure / rainure,
et **en ce que** les moyens presseurs (5) comportent une embase (53) qui porte le panneau d'appui (51) et qui est munie d'un second élément (521) dudit couple nervure / rainure.

6. Ancillaire pour chirurgie osseuse, selon la revendication 5, **caractérisé en ce que** l'embase (53) des moyens presseurs (5) comporte un panneau inférieur (531), en regard d'une face inférieure (34) dudit corps de visée (3),
lequel panneau inférieur (531) comporte deux extrémités :
- une première extrémité (532) munie du panneau d'appui (51), et
- une seconde extrémité (533) munie des moyens de guidage en translation (52).

7. Ancillaire pour chirurgie osseuse, selon l'une quelconque des revendications 2 à 6, **caractérisé en ce que** les moyens presseurs (5) comportent des moyens de préhension (55), avantageusement portés par ladite embase (53) selon la revendication 5, pour la manoeuvre en translation par un opérateur dudit panneau d'appui (51).

8. Ancillaire pour chirurgie osseuse, selon l'une quelconque des revendications 2 à 7, **caractérisé en ce que** les moyens de guidage en translation (331, 52) comportent des moyens d'indexation (5211, 3311), pour conférer un pas en translation audit panneau d'appui (51).

9. Ancillaire pour chirurgie osseuse, selon la revendication 8, **caractérisé en ce que** les moyens presseurs (5) comportent des moyens (5513) pour inactiver lesdits moyens d'indexation (5211, 3311), en particulier pour une manoeuvre en écartement dudit panneau d'appui (51) par rapport à la face frontale avant (31) dudit corps de visée (3).

10. Ancillaire pour chirurgie osseuse, selon l'une quelconque des revendications 1 à 9, **caractérisé en ce que** les moyens presseurs (5) sont amovibles par rapport audit ancillaire (1).

11. Ancillaire pour chirurgie osseuse, selon l'une quelconque des revendications 1 à 10, **caractérisé en ce que** l'organe palpeur (4) coopère avec ledit corps de visée (3) par le biais de moyens de réglage en hauteur (45), selon un second axe de translation (A) orienté perpendiculairement à l'axe (37') dudit au moins un orifice de visée (37).

12. Ancillaire pour chirurgie osseuse, selon l'une quelconque des revendications 1 à 11, **caractérisé en ce que** le corps de visée (3) comporte deux orifices traversants (37) juxtaposés et coplanaires dans un plan de visée (P).

13. Ancillaire pour chirurgie osseuse, selon la revendication 12, **caractérisé en ce que** ladite au moins une poignée (2) s'étend dans un plan (L) perpendiculaire, ou au moins approximativement perpendiculaire, audit plan de visée (P).

14. Système d'ancillaires, adapté à être couplé à une arthroscopie, comprenant :
(i) au moins un premier ancillaire (1) selon l'une quelconque des revendications 1 à 13,
(ii) au moins un second ancillaire (6) du type préhenseur / tournevis,
lequel au moins un second ancillaire (6) comprend :
- une portion amont (61), destinée à pénétrer dans ledit orifice de visée (37) du corps de visée (3) et à faire saillie au niveau de ladite face frontale avant (31), qui comporte une extrémité amont (611) formant tête de forage / vissage (6111) et un profil de filet progressif (6112), et
- une portion aval (62), destinée à coopérer avec un organe de manoeuvre en rotation,
- une structure de butée (63) ménagée entre lesdites portions amont (61) et aval (62), adaptée à venir en butée contre la face frontale arrière (32) du corps de visée (3), et
- un canal central (64), agencé coaxialement audit second ancillaire (6), adapté au passage d'une broche (7).

15. Système d'ancillaires selon la revendication 14, **caractérisé en ce qu'**il comprend encore une réglette (8) comportant :
- une première partie de mesure (811) munie d'une graduation (8111), adaptée à mesurer la profondeur d'enfoncement d'une broche (7) dans le fragment osseux (F) et la surface osseuse cible (S),
- une seconde partie de mesure (812) munie d'une graduation, adaptée à mesurer la largeur du fragment osseux (F), et
- une partie d'assemblage (82) avec le corps de visée (3), au niveau de sa face frontale arrière (32).

## Patentansprüche

1. Hilfsmittel für Knochenchirurgie, vorteilhafterweise für operative Arthroskopie, das zum Positionieren und zum Befestigen eines Knochenstücks an einer Zielknochenoberfläche ausgelegt ist,
wobei das Hilfsmittel (1)
(i) mindestens einen Betätigungsgriff (2),
(ii) einen Körper zum Zielen (3) mit - zwei entgegengesetzten Frontseiten, einer vorderen (31) und einer hinteren (32), und - mindestens einem Loch zum Zielen (37), das an den beiden Frontseiten (31, 32) mündet, zum Führen mindestens einer Schraube und/oder Nadel, die dazu bestimmt ist, in das Knochenstück (F) und in die Zielknochenoberfläche (F) eingebracht zu werden,
(iii) ein Tastorgan (4), das sich auf der vorderen Frontseite (31) des Körpers zum Zielen (3) erhaben erstreckt und eine Unterseite (411) aufweist, die dazu bestimmt ist, auf einem Teil der Zielknochenoberfläche (S) aufzuliegen, wobei die vordere Frontfläche (31) des Körpers zum Zielen (3) und die Unterseite (411) des Tastorgans (4) gemeinsam eine diedrische Aufnahmefläche (D) bilden, an der sich des Knochenstück (F) abstützen soll,
aufweist,
**dadurch gekennzeichnet, daß** das Hilfsmittel (1) Druckmittel (5) aufweist, die zum zeitweiligen Andrücken des Knochenstücks (F) an der diedrischen Aufnahmefläche (D) ausgelegt sind.

2. Hilfsmittel für Knochenchirurgie gemäß Anspruch 1, **dadurch gekennzeichnet, daß** die Druckmittel (5)
- eine Druckplatte (51), die gegenüber der vorderen Frontseite (31) des Körpers zum Zielen (3) angeordnet ist, und
- Mittel (52) zum translatorischen Führen, die dazu ausgelegt sind, die Druckplatte (51) entlang einer zum mindestens einen Loch zum Zielen (37) des Körpers (3) parallel ausgerichteten Translationsachse (T) zu führen, um das Regeln von deren Abstand zu der vorderen Frontseite (31) des Körpers zum Zielen (3) sicherzustellen,
aufweisen.

3. Hilfsmittel für Knochenchirurgie gemäß Anspruch 2, **dadurch gekennzeichnet, daß** die Mittel (52) zum translatorischen Führen der Druckmittel (5) mit den Mitteln (331) zum translatorischen Führen des Körpers zum Zielen (3) zusammenwirken.

4. Hilfsmittel für Knochenchirurgie gemäß Anspruch 3, **dadurch gekennzeichnet, daß** die Druckmittel (5) und der Körper zum Zielen (3) mittels mindestens eines Paars Rippe/Rille (331, 521) zusammenwirken.

5. Hilfsmittel für Knochenchirurgie gemäß Anspruch 4, **dadurch gekennzeichnet, daß** der Körper zum Zielen (3) zwei Seitenflächen (33) aufweist, von denen jede ein erstes Element (331) des Paars Rippe/Rille aufweist und
daß die Druckmittel (5) eine Basis (53) aufweisen, die die Druckplatte (51) trägt und die mit einem zweiten Element (521) des Paars Rippe/Rille versehen ist.

6. Hilfsmittel für Knochenchirurgie gemäß Anspruch 5, **dadurch gekennzeichnet, daß** die Basis (53) der Druckmittel (5) eine einer Unterseite (34) des Körpers zum Zielen (3) gegenüberliegende untere Platte (531) aufweist,
wobei die untere Platte (531) zwei Enden aufweist:
- ein mit der Druckplatte (51) versehenes erstes Ende (532) und
- ein mit den Mitteln (52) zum translatorischen Führen versehenes zweites Ende (533).

7. Hilfsmittel für Knochenchirurgie gemäß einem der Ansprüche 2 bis 6, **dadurch gekennzeichnet, daß** die Druckmittel (5) Greifmittel (55) für das translatorische Bewegen der Druckplatte (51) durch einen Bediener aufweisen, die vorteilhafterweise von der Basis (53) gemäß Anspruch 5 getragen sind.

8. Hilfsmittel für Knochenchirurgie gemäß einem der Ansprüche 2 bis 7, **dadurch gekennzeichnet, daß** die Mittel (331, 52) zum translatorischen Führen Indexierungsmittel (5211, 3311) aufweisen, um der Druckplatte (51) einen Translationsschritt zu verleihen.

9. Hilfsmittel für Knochenchirurgie gemäß Anspruch 8, **dadurch gekennzeichnet, daß** die Druckmittel (5) Mittel (5513) zum Deaktivieren der Indexierungsmittel (5211, 3311), insbesondere für eine Betätigung der Druckplatte (51) zum Ändern des Abstands von der vorderen Frontseite (31) des Körpers zum Zielen (3) aufweisen.

10. Hilfsmittel für Knochenchirurgie gemäß einem der Ansprüche 1 bis 9, **dadurch gekennzeichnet, daß** die Druckmittel (5) vom Hilfsmittel (1) abnehmbar sind.

11. Hilfsmittel für Knochenchirurgie gemäß einem der Ansprüche 1 bis 10, **dadurch gekennzeichnet, daß** das Tastorgan (4) mit dem Körper zum Zielen (3) über Mittel (45) zum Einstellen der Höhe entlang einer zweiten Translationsachse (A) zusammenwirkt, die zur Achse (37') des mindestens einen Lochs zum Zielen (37) quer ausgerichtet ist.

12. Hilfsmittel für Knochenchirurgie gemäß einem der Ansprüche 1 bis 11, **dadurch gekennzeichnet, daß** der Körper zum Zielen (3) zwei nebeneinander angeordnete und in einer Ebene (P) zum Zielen koplanare Durchgangslöcher (37) aufweist.

13. Hilfsmittel für Knochenchirurgie gemäß Anspruch 12, **dadurch gekennzeichnet, daß** sich der mindestens eine Griff (2) in einer zur Ebene (P) zum Zielen senkrechten oder mindestens näherungsweise senkrechten Ebene (L) erstreckt.

14. Hilfsmittelsystem, das dazu ausgelegt ist, mit einer Arthroskopie gekoppelt zu werden, und
(i) mindestens ein erstes Hilfsmittel (1) gemäß einem der Ansprüche 1 bis 13,
(ii) mindestens ein zweites Hilfsmittel (6) vom Typ eines Greifers/Schraubendrehers
aufweist,
wobei das mindestens eine zweite Hilfsmittel (6)
- einen vorneliegenden Abschnitt (61), der dazu bestimmt ist, in das Loch zum Zielen (37) einzudringen und an der vorderen Frontseite (31) hervorzustehen, und der ein einen Bohrkopf/Schraubkopf bildendes vorderes Ende (611) und ein progressives Schraubgewinde (6112) aufweist, und
- einen rückwärtigen Abschnitt (62), der dazu bestimmt ist, mit einem Organ zur Drehbetätigung zusammenzuwirken,
- eine Zielstruktur (63), die zwischen dem vorneliegenden und dem rückwärtigen Abschnitt angeordnet ist und dazu ausgelegt ist, an der hinteren Frontseite (32) des Körpers zum Zielen (3) in Anschlag zu gehen, und
- einen zentralen Kanal (64), der zum zweiten Hilfsmittel (6) koaxial angeordnet ist und für das Hindurchführen einer Nadel ausgelegt ist,
aufweist.

15. Hilfsmittelsystem gemäß Anspruch 14, **dadurch gekennzeichnet, daß** es außerdem eine Leiste (8) mit
- einem mit einer Graduierung (8111) versehenen ersten Meßteil (811), das zum Messen einer Eindringtiefe einer Nadel (7) in das Knochenstück (F) und in die Zielknochenoberfläche (S) ausgelegt ist,
- einem mit einer Graduierung versehenen zweiten Meßteil (812), das zum Messen der Breite des Knochenstücks (F) ausgelegt ist, und
- einem Teil (82) zum Zusammenfügen mit dem Körper zum Zielen (3) an dessen rückwärtiger Frontseite (32)
aufweist.

## Claims

1. An ancillary for bone surgery, advantageously for operative arthroscopy, operable for positioning and fixing a bone fragment on a target bone surface,
wherein said ancillary (1) comprises:
(i) at least one operating handle (2),
(ii) a sighting body (3) comprising - two opposite end faces, a front one (31) and a rear one (32), and - at least one sighting hole (37) that opens to said two end faces (31, 32), for guiding at least one screw and/or pin intended to be implanted into said bone fragment (F) and into said target bone surface (S),
(iii) a feeler element (4), protruding from the front end face (31) of said sighting body (3) and having a lower face (411) intended to bear against a part of said target bone surface (S),
wherein said front end face (31) of said sighting body (3) and said lower face (411) of said feeler element (4) form together a dihedral receiving surface (D) against which said bone fragment (F) is intended to bear,
**characterized in that** said ancillary (1) comprises pressing means (5) operable for temporarily holding said bone fragment (F) bearing against said dihedral receiving surface (D).

2. The ancillary for bone surgery according to claim 1, **characterized in that** the pressing means (5) comprise:
- a bearing panel (51) arranged opposite the front end face (31) of said sighting body (3), and
- translational guiding means (52), for guiding said bearing panel (51) in translation along a translation axis (T) directed parallel to said at least one sighting hole (37) of the sighting body (3) so as to ensure its spacing adjustment with respect to said front end face (31) of the sighting body (3).

3. The ancillary for bone surgery according to claim 2, **characterized in that** the translational guiding means (52) of the pressing means (5) cooperate with translational guiding means (331) of said sighting body (3).

4. The ancillary for bone surgery according to claim 3, **characterized in that** the pressing means (5) and the sighting body (3) cooperate through at least one rib / groove couple (331, 521).

5. The ancillary for bone surgery according to claim 4, **characterized in that** the sighting body (3) has two lateral faces (33) each comprising a first element (331) of said rib / groove couple,
and **in that** the pressing means (5) comprise a base (53) that carries the bearing panel (51) and that is provided with a second element (521) of said rib / groove couple.

6. The ancillary for bone surgery according to claim 5, **characterized in that** the base (53) of the pressing means (5) comprises a lower panel (531), opposite a lower face (34) of said sighting body (3),
wherein said lower panel (531) comprises two ends:
- a first end (532) provided with the bearing panel (51), and
- a second end (533) provided with translational guiding means (52).

7. The ancillary for bone surgery according to any one of claims 2 to 6, **characterized in that** the pressing means (5) comprise gripping means (55), advantageously carried by said base (53) according to claim 5, for the translational operation of said bearing panel (51) by an operator.

8. The ancillary for bone surgery according to any one of claims 2 to 7, **characterized in that** the translational guiding means (331, 52) comprise indexing means (5211, 3311), for providing said bearing panel (51) with a translational pitch.

9. The ancillary for bone surgery according to claim 8, **characterized in that** the pressing means (5) comprise means (5513) for deactivating said indexing means (5211, 3311), in particular for operating said bearing panel (51) so as to space it from the front end face (31) of said sighting body (3).

10. The ancillary for bone surgery according to any one of claims 1 to 9, **characterized in that** the pressing means (5) are removable with respect to said ancillary (1).

11. The ancillary for bone surgery according to any one of claims 1 to 10, **characterized in that** the feeler element (4) cooperates with said sighting body (3) through height adjustment means (45), along a second translation axis (A) directed perpendicular to the axis (37') of said at least one sighting hole (37).

12. The ancillary for bone surgery according to any one of claims 1 to 11, **characterized in that** the sighting body (3) comprises two through-holes (37) juxtaposed and coplanar to each other in a sighting plane (P).

13. The ancillary for bone surgery according to claim 12, **characterized in that** said at least one handle (2) extends in a plane (L) perpendicular, or at least approximately perpendicular, to said sighting plane (P).

14. A system of ancillaries, capable of being coupled to an arthroscopy, comprising:
(i) at least one first ancillary (1) according to any one of claims 1 to 13,
(ii) at least one second ancillary (6) of the gripper / screwdriver type,
wherein said at least one second ancillary (6) comprises:
- an upstream portion (61), intended to enter said sighting hole (37) of the sighting body (3) and to protrude at said front end face (31), which comprises an upstream end (611) forming a drilling / screwing head (6111) and a progressive thread profile (6112), and
- a downstream portion (62), intended to cooperate with a rotational operation member,
- an abutment structure (63) arranged between said upstream (61) and downstream (62) portions, adapted to come into abutment against the rear end face (32) of the sighting body (3), and
- a central channel (64), arranged coaxially to said second ancillary (6), adapted to the passage of a pin (7).

15. The system of ancillaries according to claim 14, **characterized in that** it also comprises a ruler (8) having:
- a first measurement portion (811) provided with a scaling (8111), for measuring the depth of penetration of a pin (7) into the bone fragment (F) and the target bone surface (S),
- a second measurement portion (812) provided with a scaling, for measuring the width of the bone fragment (F), and
- a portion (82) for the assembly to the sighting body (3), at its rear end face (32).
